# EUROPEAN PATENT SPECIFICATION

(11) **EP 4 027 822 B1**
(45) Date of publication and mention of the grant of the patent: **27.11.2024**
(21) Application number: 20772371.9
(22) Date of filing: 08.09.2020
(51) Int. Cl.: A41C 3/00, A41D 1/00, A41D 27/20

(54) **BRA, GARMENT AND METHOD**
BÜSTENHALTER, KLEIDUNGSSTÜCK UND VERFAHREN
SOUTIEN-GORGE, VÊTEMENT ET PROCÉDÉ

(30) Priority: 09.09.2019 GB 201912928
(43) Date of publication of application: 20.07.2022
(73) Proprietor: Prevayl Innovations Limited, Wilmslow SK9 1LD (GB)
(72) Inventor: HEPWORTH, Isabel Rose, Wilmslow Cheshire SK9 3NR (GB)
(74) Representative: Appleyard Lees IP LLP
(86) International application number: PCT/GB2020/052156
(87) International publication number: WO 2021/048534

(56) References cited:
- WO-A1-2018/206853
- CN-A- 104 997 176
- CN-A- 104 997 176
- CN-A- 105 266 765
- CN-U- 205 005 938
- CN-U- 205 005 938
- CN-U- 205 285 029
- CN-U- 205 285 029
- US-A1- 2018 243 549
- US-A1- 2019 201 772

## Description

### Cross-Reference to Related Applications

This application claims priority from United Kingdom Patent Application number 1912928.7 filed on 9 September 2019.

### Background

The present invention relates to a bra for use in measuring biosignals of a wearer, a garment comprising such a bra, and a method of manufacture.

Document US 2019/201772 A1 discloses a bra for use in measuring biosignals of a wearer according to the preamble of claim 1.

Garments incorporating sensors are wearable electronics used to measure and collect information from a wearer. Such garments are commonly referred to as 'smart clothing'. It is advantageous to measure biosignals of the wearer during exercise and garments designed for such a purpose are generally close-fitting.

It is known to provide a bra, sports bra, or other close-fitting top-like garment to which an electronic device (i.e. an electronics module, or related components) is attached in a prominent position, such as on the chest or between the shoulder blades. Whilst this positioning may allow the device to be readily accessed, it can be both uncomfortable for the wearer and unsightly.

It is an object of the present invention to provide an improved bra for use in measuring biosignals of a wearer and/or to address one or more of the problems discussed above, or discussed elsewhere, or to at least provide an alternative bra.

### Summary

According to the present invention there is provided a bra, garment and method as set forth in the appended claims. Other features of the invention will be apparent from the dependent claims, and the description which follows.

According to a first aspect of the present invention there is provided a bra for use in measuring biosignals of a wearer according to independent claim 1, the bra comprising: a front region; a rear region; a pair of side regions extending between the front region and the rear region; and a measuring apparatus comprising a sensor assembly comprising one or more sensors, wherein one of the side regions is arranged to receive an electronics module. The electronics module is arranged to receive measurement signals from the one or more electronics modules.

The electronics module is located in one of the side-regions. The electronics module may be part of the measuring apparatus.

Such a construction enables the measuring apparatus to be provided in a position which is both readily accessible and unobtrusive, whilst ensuring wearer comfort. Also, this means that functionality (e.g. support or opening-closing configurations) or aesthetics (look or feel) of the front and rear regions of the bra do not have to be compromised to accommodate the measuring apparatus.

The term "bra" otherwise known as "brassiere" refers to a form-fitting garment designed to support or cover the wearer's breasts. Bras are typically used as undergarments but may be provided as or incorporated into outerwear. Bras may be built into garments such as swimsuits, camisoles, and dresses and in particular backless dresses.

The front, rear and side regions may be together configured to extend circumferentially about the wearer when the bra is in the as-worn configuration. The regions may extend circumferentially about the torso of the wearer.

The electronics module may be located in a mounting arrangement provided in one of the side regions. The mounting arrangement may comprise a pocket provided in one of the side regions. The pocket may be a hidden pocket. The inside of the pocket may be accessible to provide access to the electronics module. The electronics module may be removable from the pocket. The side region comprising the pocket may comprise a first layer of material and a second layer of material positioned adjacent and external to the first layer of material. The internal space of the pocket may be formed between the first layer of material and the second layer of material. The pocket may be formed from an elastically resilient material. The elastically resilient pocket can help hold the electronics module in place and restrict movement of the electronics module relative to the bra. Further, the elastically resilient pocket can help urge the electronics module towards the sensor assembly or a conductive pathway therefore to form, improve or maintain a conductive or wireless communication between the electronics module and the sensor assembly.

The one or more sensors may be releasably connectable to the electronics module. This facilitates removal of the electronics module from its location in one of the side regions. In particular, this facilitates removal from the mounting arrangement/pocket. This may be necessary or desirable, for example, for washing the bra or maintenance/battery replacement of the electronics module.

The bra comprises an underband. The underband may form part of the front region, rear region and pair of side regions. The underband extends below a lower edge of the front region. The underband extends below a lower edge of the rear region. The underband extends below a lower edge of the side regions. The underband may be configured to extend circumferentially around the wearer when the bra is in the as-worn configuration. The one or more of the sensors are displaced from the underband. The sensors being displaced from the underband means that all of the sensors are not between the underband and the wearer's skin when the bra is in the as-worn configuration. The sensors being displaced from the underband may mean that a part or all of the sensors are not in contact with the underband. In other words, the sensors are provided on the underband but are provided on one of the side regions of the bra. In existing devices, sensors are typically provided on the underband to help ensure that the sensors are held in tight contact with the skin. A downside of this approach is that the underband needs to be wide in order to ensure that a sufficient surface area is provided for the sensors. This means that conventional bras for biosensing have a wider underband than conventional bras. A wider underband is uncomfortable for the wearer, especially during prolonged user or exercises. Advantageously, it has been found that positioning the sensors in other regions of the bra and in the side region, in particular, ensures sufficient skin sensor contact while avoiding the need to provide an unnecessarily wide underband. In addition, a far larger sensor surface area can be provided than in existing bras for biosensing. The underband may therefore be a narrow underband. This increases bra comfort during exercise. In other words, the underband does not now need to be unnecessarily wide, to accommodate sensors.

All of the sensor assembly is located in the side region. That is, one or more of the sensors are located in the side region. The side region refers to the same side region as where the electronics module is located. As the electronics module is located in the same side region, providing the sensor assembly in the side region ensures short distances between sensors and the electronics module. In other words, as the sensors and electronics module are located proximate to one another in the same side region, the communication distance between the electronics module and the sensors is reduced. The communication pathway, such as a conductor or an air interface, is less susceptible to noise and interference from external sources. This ensures that signal quality is high, as signal noise is low. Such a construction therefore facilitates high quality data collection.

The front region may comprise a front panel and the rear region may comprise a rear panel. The front panel may extend into the side region. The rear panel may extend into the side region. The electronics module may be provided on a panel or panels in the side region. The facilitates simple construction. The electronics module may be provided in a panel or panels in the side region. This facilitates a layered panel construction, or one wherein the electronics module is encapsulated in a panel or panels. This is advantageous as it provides protection to, and secure retention of, the module when the bra is in use. The panel or panels in the side region may be the front panel and/or the rear panel. The front and rear panels may connect at the side.

The side region may comprise a side panel. The side panel may be a side overlay panel. The side overlay panel may overlay a panel or panels provided in the side region. Alternatively, the side panel may connect a panel or panels, such as the front and rear panel. The electronics module may be provided on or in the side panel.

The different panels may comprise separate constructions that are joined together at one or more seam lines using connecting technologies such as stitching, bonding, welding, adhesives, and the like. Alternatively, the different panels comprise integral extensions of one another. For instance, a knitting or weaving process may be used to seamlessly and integrally knit or weave the different panels to form the bra. Alternatively, one or more of the panels are seamlessly and integrally knit or woven with another region while remaining panels comprise separate constructions that are connected to the integrally knit or woven portions using connecting technologies described herein.

At least a part of the sensor assembly is provided on an inner panel surface of a panel or panels in the side region. The part of the sensor assembly provided on the inner panel surface may comprise tracking and/or the electrodes. The electronics module may be provided on an outer panel surface. The outer panel surface may be the outer surface of a panel or panels in the side region. The sensors may be skin contact sensors.

The electronics module and the sensor assembly may be electrically connected. This facilitates a stable and secure connection between the electronics module and the sensor assembly. Additionally, or alternatively, the electronics module and the sensor assembly may be wirelessly connected.

The sensor assembly may comprise electrodes. Such electrodes enable the sensor assembly to perform bioelectric and bioimpedance measurements amongst others. The electrodes may comprise concentric ring electrodes. Concentric ring electrodes provide improved spatial resolution. The sensor assembly may comprise conductive pads. The sensor assembly may comprise blob electrodes. Blob electrodes comprise a blob of conductive material. Blob electrodes provide high signal amplitude. The electrodes may comprise a conductive transfer. Conductive transfers are advantageous as they are lightweight, flexible and can withstand high temperatures. The conductive transfer layer may comprise conductive layer sandwiched between an outwardly-facing layer and an inwardly-facing layer. The conductive layer may comprise silver and graphene ink. The electrodes may comprise conductive threads. The sensors may be connected to the electronics module by a conductive transfer. Additionally, or alternatively, the sensors may be connected to the electronics module by conductive threads.

The bra may comprise a communicator. The communicator may be part of the electronics module or may be separate to the electronics module. The communicator may be arranged to transmit data to a remote electronic device via a wireless network which may be a mobile network. The communicator may be any form of communicator operable to communicate data wirelessly via one or more base stations. The communicator may provide wireless communication capabilities for the bra and may enable the bra to communicate via one or more wireless communication protocols such as used for communication on: a wireless wide area network (WWAN), a wireless metroarea network (WMAN), a wireless local area network (WLAN), a wireless personal area network (WPAN), a near field communication (NFC), and a cellular communication network. The cellular communication network may be a fourth generation (4G) LTE, LTE Advanced (LTE-A), fifth generation (5G), sixth generation (6G), and/or any other present or future developed cellular wireless network. The communicator may comprise a 5G communications chip and/or a Bluetooth Low Energy chip. This facilitates fast and/or efficient communication with the electronics module for data upload, update or calibration.

The measuring assembly may be configured to make one or more of the following measurements: electrocardiography; bioelectricity; bioimpedance. These are typically useful measurements in this field. More generally, any biosignal may be measured.

According to a second aspect of the present invention there is provided a garment comprising a bra according to the first aspect and a top provided over the bra.

The second aspect of the present invention comprises all of the features of the first aspect of the present invention.

The bra may be attached to the top at the shoulders.

According to a third aspect of the present invention there is provided a method of manufacturing a bra according to the first aspect, the method comprising the steps of: providing a bra comprising: a front region; a rear region; a pair of side regions extending between the front region and the rear region; and a measuring apparatus comprising a sensor assembly comprising one or more sensors.

The method may comprise positioning an electronics module of the measurement apparatus on or in one of the side regions.

The method may further comprise the step of: providing a pocket on or in one of the side regions in which an electronics module of the measuring apparatus is to be located. The electronics module is arranged to receive measurement signals from the one or more electronics modules. The pocket may be an elastically resilient pocket. The pocket may be a hidden pocket. The electronics module may be located in the pocket. The method may comprise the step of providing a bra and providing a top over the bra. The bra may be attached to the top at the shoulders.

### Brief Description of the Drawings

For a better understanding of the invention, and to show how embodiments of the same may be carried into effect, reference will now be made, by way of example only, to the accompanying diagrammatic Figures in which:
Figures 1 - 3 show a front, rear and side view respectively of a bra according to the present invention;
Figures 4-6 show a front, rear and side view respectively of the bra of Figures 1 - 3 with the pocket, side overlay panel and front overlay panel omitted;
Figures 7 - 9 show a front, rear and side view respectively of a garment comprising a bra according to the present invention; and
Figure 10 shows general methodology associated with the present invention, in terms of manufacture of a bra according to example embodiments.

### Detailed Description

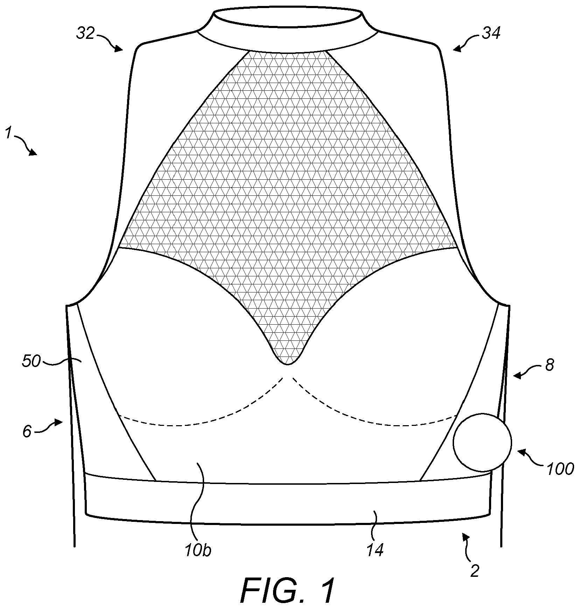

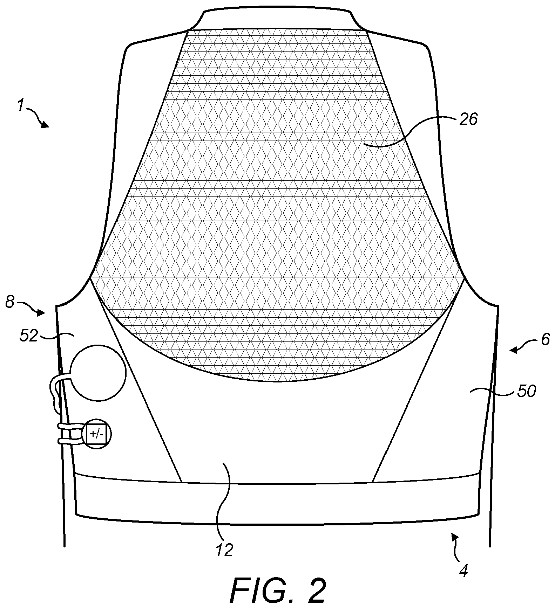

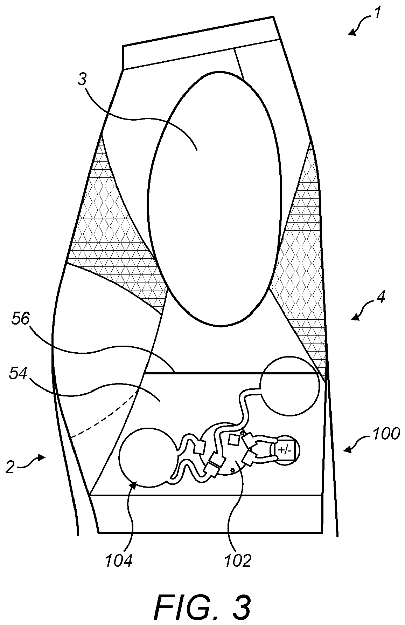

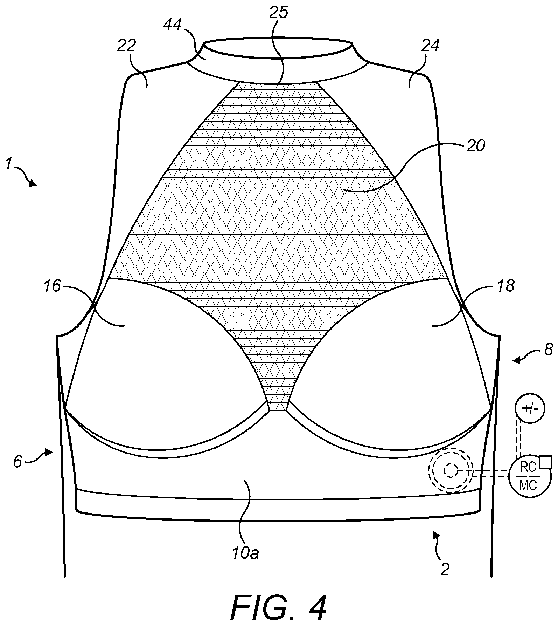

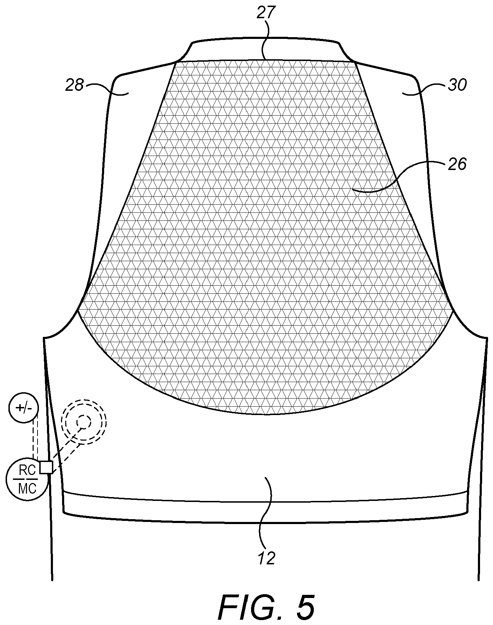

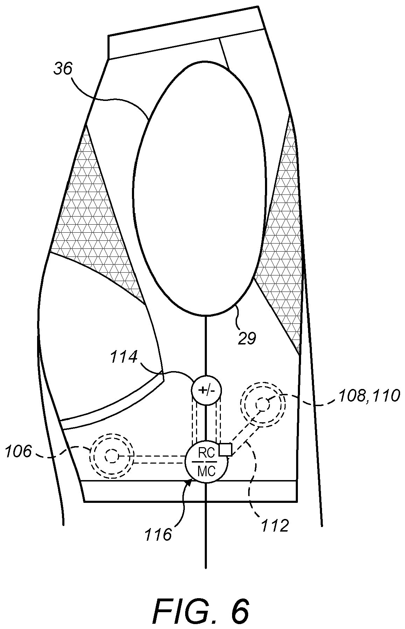

Referring to Figures 1 - 6, there is shown a bra 1. The bra 1 comprises a front region 2, a rear region 4, and a pair of side regions 6, 8 extending between the front region 2 and the rear region 4. The front 2, rear 4 and side regions 6, 8 are together configured to extend circumferentially around the torso of the wearer when the bra 1 is in the as-worn configuration. The front region 2 comprises a front underlay panel 10a and the rear region 4 comprises a rear panel 12. The front underlay panel 10a and rear panel 12 are both formed from a breathable knit fabric material. The front underlay panel 10a and rear panel 12 extend into the side regions 6, 8 to connect at either side of the bra 1. The lower edge of the front underlay panel 10a and the lower edge of the rear panel 12 are parallel and vertically aligned. A narrow underband 14 extends below the lower edge of the front underlay panel 10a and rear panel 12.

The front region 2 further comprises a pair of breast-contacting surfaces in the form of moulded cups 16, 18, a sternal panel 20 formed of a breathable mesh material, a pair of front shoulder strap portions 22, 24 and a front overlay panel 10b. As can be seen from Figures 1 - 3, the front overlay panel 10b is arranged to overlay the moulded cups 16, 18 and the medial region of the front underlay panel 10a. The sternal panel 20 extends laterally between the medial edges of the front shoulder strap portions 22, 24, and downwardly from an upper edge 25 to the upper edge of the front overlay panel 10b. In this exemplary embodiment, the front shoulder strap portions 22, 24 are formed integrally with the front underlay panel 10a. Nevertheless, the person skilled in the art will appreciate that the front shoulder strap portions 22, 24 may be formed separately and subsequently connected to the front underlay panel 10b.

The rear region 4 further comprises an upper back panel 26 formed of a breathable mesh material and a pair of rear shoulder strap portions 28, 30. The upper back panel 26 extends laterally between the medial edges of the rear shoulder strap portions 28, 30 and downwardly from an upper edge 27 to the upper edge of the rear panel 12. The front and rear shoulder strap portions 22, 24, 28, 30 connect over the shoulders to form shoulder straps, shown generally at 32 and 34. The shoulder straps 32, 34 widen toward the shoulder. The lateral edges of the shoulder straps 36 and upper corners 29 of the rear panel 12 define armholes 3. At the shoulders, the shoulder straps 32, 34 each comprise a rounded cut-out, which, together with the upper edge 25 of the sternal panel and the upper edge 27 of the upper back panel, define a neckline.

A funnel collar 44 is attached about the neckline and configured to extend circumferentially around the neck of the wearer when the bra is in the as-worn configuration. The collar 44 and armholes 3 have a relaxed fit to avoid irritating the wearer.

Each side region 6, 8 comprises a side overlay panel 50, 52. Each side overlay panel 50, 52 overlays the front and rear panels 10, 12 in the region below the armholes 3, and extend around the side regions 6, 8 to project slightly onto the front and rear regions 2, 4. Each side overlay panel 50, 52 extends downwardly from the lower edge of the respective armhole to the upper edge of the underband 14, and the side overlay panels 50, 52 widen toward the underband 14.

A pocket 54 is provided on the left side overlay panel 52. The pocket 54 extends between the widening edges of the side overlay panel 52 at a height of approximately 3/5 of the height of the side overlay panel 52. A horizontal top opening 56 at the top edge of the pocket 54 provides access therein.

The bra 1 is for use in measuring biosignals of a wearer and further comprises a measuring apparatus 100 comprising an electronics module 102 and a sensor assembly 104 comprising three sensors. In this exemplary embodiment, the three sensors are two concentric ring electrodes 106, 108 and a blob electrode 110.

The electrodes 106, 108, 110 are formed of conductive transfers or conductive threads, which are also suitable for use as tracking between the electrodes and the electronics module. Conductive transfers comprise a conductive layer, comprising a mixture of silver and graphene ink (or other conductive ink), sandwiched between an inwardly-facing layer and an outwardly-facing layer. Where conductive transfers are used as tracking, the inwardly-facing layer is arranged such that, when the bra is in use, the inwardly-facing layer faces the skin of the wearer. Areas of the conductive layer can be exposed by forming inwardly-facing layer in sections such that the inwardly-facing layer has openings which expose areas of the conductive layer, and the electrodes are formed from the exposed areas of the conductive layer. Conductive transfers are described in more detail in GB 2555592 B.

The electrodes 106, 108, 110 are arranged to contact the wearer's skin when the bra 1 is in use. In this exemplary embodiment, the electrodes 106, 108, 110 are displaced from being directly beneath the underband 14 (that is, they are not provided between the underband 14 and the wearer's skin - in other words, the electrodes 106, 108, 110 are not contacted by the underband 14), and are provided in the vicinity of the electronics module 102. Providing the electrodes 106, 108, 110 at a location displaced from the underband 14 increases comfort and allows for electrodes 106, 108 with a larger surface area to be used without requiring an increased width of underband 14. Nevertheless, the electrodes 106, 108, 110 remain secure by the applied pressure from the panels 10a, 12, 52. Additionally, providing the electrodes 106, 108, 110 in the vicinity of the electronics module 102 helps to reduce collected signal noise as the communication distance between the electronics module 102 and the electrodes 106, 108, 110 is reduced.

The electronics module 102 is provided in the pocket 54. Tracking 112 extends from the electrodes 106, 108, 110 to a position adjacent the electronics module 102. A connection is formed between the tracking 112 and the electronics module 102 through the panels 10a, 12, 52 and into the pocket 54. To enable the connection to be made, areas of the conductive layer are exposed by removing areas of the outwardly-facing layer. A conductive connection, such as a pogo pin or conductive thread, releasably connects the conductive layer to the electronics module 102. The tracking 112 being releasably connected the electronics module 102 enables the electronics module 102 to be disconnected from the tracking 112 and removed from the pocket 54 through the opening 56 where necessary or desirable, for example for washing or battery replacement.

The electronics module 102 comprises a power source 114 and, shown generally at 116, a processor, a 5G communications chip and a Bluetooth Low Energy (BLE) chip. The electronics module 102 is appropriately programmed (which include the module being programmable, or the program being embedded) to perform the functions described herein. The 5G communications chip and/or BLE chip enable information to be transmitted from the electronics module 102 for subsequent processing or analysis. The electronics module 102 and the sensor assembly comprising electrodes 106, 108, 110 are configured to measure electrocardiography, bioelectricity and bioimpedance. Such particular measurements are informative in monitoring health and fitness of a wearer, particularly during exercise.

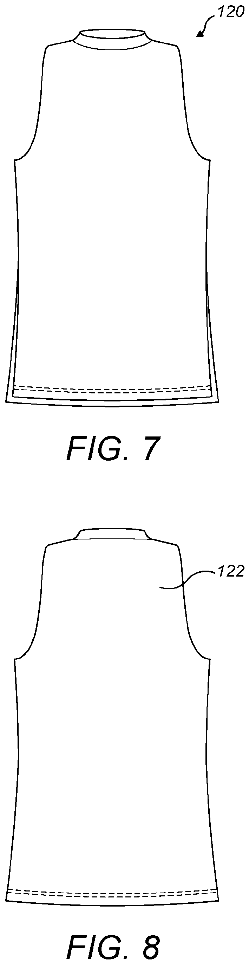

Turning now to Figures 7 - 9, a garment 120 is shown comprising a top provided over a bra 1 according to the present invention. In this exemplary embodiment, the top is a loose-fitting sleeveless top 122 comprising an opening 124 at the side hem 126. The top 122 is attached to the bra 1 along the shoulder line 128 either side of the collar 44 such that the top is able to move freely relative to the bra and the bra does not pull on the top when the garment is in an as-worn configuration.

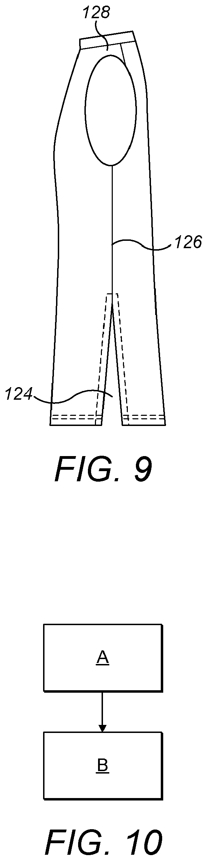

In Figure 10, general methodology principles are shown. Step A comprises providing a bra comprising: a front region; a rear region; a pair of side regions extending between the front region and the rear region; and a measuring apparatus comprising an electronics module and one or more sensors. Step B comprises the step of positioning the electronics module in one of the side regions.

In summary, there is provided a bra, garment and method. The bra comprises: a front region; a rear region; a pair of side regions extending between the front region and the rear region; and a measuring apparatus comprising an electronics module and one or more sensors. The electronics module is located on one of the side regions.

Although a few preferred embodiments of the present invention have been shown and described, it will be appreciated by those skilled in the art that various changes and modifications might be made without departing from the scope of the invention, as defined in the appended claims.

The preceding description with reference to the accompanying drawings is provided to assist in a comprehensive understanding of various embodiments of the disclosure as defined by the claims. It includes various specific details to assist in that understanding but these are to be regarded as merely exemplary. Accordingly, those of ordinary skill in the art will recognize that various changes and modifications of the various embodiments described herein can be made within the scope of the claims. In addition, descriptions of well-known functions and constructions may be omitted for clarity and conciseness.

The terms and words used in the preceding description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. The terms "front", "rear", "side", "upper", "lower", "over", "under", "inner", "outer", "medial", "lateral" and like terms are used to refer to the bra and its components in the orientation in which it is illustrated, which is the orientation in which it is intended to be used but should not be taken as otherwise limiting. Like reference numerals are used to denote like features throughout the drawings, which are not to scale.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

## Claims

1. A bra (1) for use in measuring biosignals of a wearer, the bra (1) comprising: a front region (2); a rear region (4); a pair of side regions (6, 8) extending between the front region (2) and the rear region (4); an underband (14) extending below a lower edge of the front region (2), rear region (4) and side regions (6, 8), and a measuring apparatus (100) comprising a sensor assembly (104) comprising one or more skin contact sensors (106, 108, 110), wherein at least a part of the one or more skin contact sensors (106, 108, 110) is provided on an inner panel surface of a panel or panels (52) in one of the side regions (6,8), **characterised in that** all of the sensor assembly (104) is provided in one of the side regions (8) and is not provided in the underband (14).

2. A bra (1) as claimed in claim 1, wherein the side region (8) where the sensor assembly (104) is provided comprises a pocket (54) in which an electronics module (102) of the measuring apparatus (100) is located.

3. A bra (1) as claimed in claim 2, wherein the pocket (54) is an elastically resilient pocket (54).

4. A bra (1) as claimed in claim 2 or 3, wherein the pocket (54) is a hidden pocket.

5. A bra (1) as claimed in any of claims 2 to 4, wherein the inside of the pocket (54) is accessible to provide access to the electronics module (102) of the measuring apparatus (100).

6. A bra (1) as claimed in claim 5, wherein the electronics module (102) is removable from the pocket (54).

7. A bra (1) as claimed in claim 5 or 6, wherein the one or more sensors (106, 108, 110) are releasably connectable to the electronics module (102).

8. A bra as claimed in any of claims 2 to 7, wherein the front region (2) comprises a front panel (10a) and the rear region (4) comprises a rear panel (12), and the front panel (10a) and/or the rear panel (2) extend into the side region (6, 8), and the pocket (54) is provided on or in the panel or panels (52) in the side region (8).

9. A bra (1) as claimed in any of claims 2 to 8, wherein the side region (8) comprises a side panel (52), and the pocket (54) is provided on or in the side panel (52).

10. A bra (1) as claimed in either of claims 8 or 9 wherein at least a part of the sensor assembly (104) is provided on an inner panel surface and the pocket (54) is provided on an outer panel surface.

11. A bra (1) as claimed in any preceding claim, wherein the measuring apparatus (100) is configured to make one or more of the following measurements: electrocardiography; bioelectricity; bioimpedance.

12. A method of manufacturing a bra (1) as claimed in any of claims 1 to 11, the method comprising the steps of: providing a bra (1) comprising: a front region (2); a rear region (4); a pair of side regions (6, 8) extending between the front region (2) and the rear region (4); an underband (14) extending below a lower edge of the front region (2), rear region (4) and side regions (6, 8); and a measuring apparatus (100) comprising a sensor assembly (104) comprising one or more skin contact sensors (106, 108, 110), wherein at least a part of the one or more skin contact sensors (106, 108, 110) is provided on an inner panel surface of a panel or panels (52) in one of the side regions (6,8); **characterised in that** all of the sensor assembly (104) is provided in one of the side regions (8) and is not provided in the underband (14).

## Patentansprüche

1. Büstenhalter (1) zur Verwendung bei der Messung von Biosignalen einer Trägerin, wobei der Büstenhalter (1) umfasst: einen vorderen Bereich (2); einen hinteren Bereich (4); ein Paar von Seitenbereichen (6, 8), die sich zwischen dem vorderen Bereich (2) und dem hinteren Bereich (4) erstrecken; einen Unterbund (14), der sich unterhalb einer Unterkante des vorderen Bereichs (2), des hinteren Bereichs (4) und der Seitenbereiche (6, 8) erstreckt, und eine Messvorrichtung (100), die eine Sensoranordnung (104) umfasst, die einen oder mehrere Hautkontaktsensoren (106, 108, 110) umfasst, wobei zumindest ein Teil des einen oder der mehreren Hautkontaktsensoren (106, 108, 110) auf einer Platteninnenfläche einer Platte oder von Platten (52) in einem der Seitenbereiche (6, 8) bereitgestellt ist, **dadurch gekennzeichnet, dass** die gesamte Sensoranordnung (104) in einem der Seitenbereiche (8) bereitgestellt ist und nicht in dem Unterbund (14) bereitgestellt ist.

2. Büstenhalter (1) nach Anspruch 1, wobei der Seitenbereich (8), in dem die Sensoranordnung (104) bereitgestellt ist, eine Tasche (54) umfasst, in der sich ein Elektronikmodul (102) der Messvorrichtung (100) befindet.

3. Büstenhalter (1) nach Anspruch 2, wobei es sich bei der Tasche (54) um eine elastisch nachgiebige Tasche (54) handelt.

4. Büstenhalter (1) nach Anspruch 2 oder 3, wobei es sich bei der Tasche (54) um eine verborgene Tasche handelt.

5. Büstenhalter (1) nach einem der Ansprüche 2 bis 4, wobei das Innere der Tasche (54) zugänglich ist, um Zugang zum Elektronikmodul (102) der Messvorrichtung (100) zu ermöglichen.

6. Büstenhalter (1) nach Anspruch 5, wobei das Elektronikmodul (102) aus der Tasche (54) entnehmbar ist.

7. Büstenhalter (1) nach Anspruch 5 oder 6, wobei der eine oder die mehreren Sensoren (106, 108, 110) lösbar mit dem Elektronikmodul (102) verbindbar sind.

8. Büstenhalter nach einem der Ansprüche 2 bis 7, wobei der vordere Bereich (2) eine vordere Platte (10a) und der hintere Bereich (4) eine hintere Platte (12) umfasst und die vordere Platte (10a) und/oder die hintere Platte (2) sich in den Seitenbereich (6, 8) erstrecken und die Tasche (54) auf oder in der Platte oder den Platten (52) im Seitenbereich (8) bereitgestellt ist.

9. Büstenhalter (1) nach einem der Ansprüche 2 bis 8, wobei der Seitenbereich (8) eine Seitenplatte (52) umfasst und die Tasche (54) auf oder in der Seitenplatte (52) bereitgestellt ist.

10. Büstenhalter (1) nach einem der Ansprüche 8 oder 9, wobei zumindest ein Teil der Sensoranordnung (104) auf einer Platteninnenfläche bereitgestellt ist und die Tasche (54) auf einer Plattenaußenfläche bereitgestellt ist.

11. Büstenhalter (1) nach einem der vorhergehenden Ansprüche, wobei die Messvorrichtung (100) so konfiguriert ist, dass sie eine oder mehrere der folgenden Messungen durchführt: Elektrokardiographie; Bioelektrizität; Bioimpedanz.

12. Verfahren zum Herstellen eines Büstenhalters (1) nach einem der Ansprüche 1 bis 11, wobei das Verfahren die folgenden Schritte umfasst: Bereitstellen eines Büstenhalters (1), umfassend: einen vorderen Bereich (2); einen hinteren Bereich (4); ein Paar von Seitenbereichen (6, 8), die sich zwischen dem vorderen Bereich (2) und dem hinteren Bereich (4) erstrecken; einen Unterbund (14), der sich unterhalb einer Unterkante des vorderen Bereichs (2), des hinteren Bereichs (4) und der Seitenbereiche (6, 8) erstreckt, und eine Messvorrichtung (100), die eine Sensoranordnung (104) umfasst, die einen oder mehrere Hautkontaktsensoren (106, 108, 110) umfasst, wobei zumindest ein Teil des einen oder der mehreren Hautkontaktsensoren (106, 108, 110) auf einer Platteninnenfläche einer Platte oder von Platten (52) in einem der Seitenbereiche (6, 8) bereitgestellt ist, **dadurch gekennzeichnet, dass** die gesamte Sensoranordnung (104) in einem der Seitenbereiche (8) bereitgestellt ist und nicht in dem Unterbund (14) bereitgestellt ist.

## Revendications

1. Soutien-gorge (1) destiné à être utilisé dans la mesure de biosignaux d'une personne le portant, le soutien-gorge (1) comprenant : une région avant (2) ; une région arrière (4) ; une paire de régions latérales (6, 8) s'étendant entre la région avant (2) et la région arrière (4) ; une sous-bande (14) s'étendant en dessous d'un bord inférieur de la région avant (2), de la région arrière (4) et des régions latérales (6, 8), et un appareil de mesure (100) comprenant un ensemble de capteurs (104) comprenant un ou plusieurs capteurs en contact avec la peau (106, 108, 110), dans lequel au moins une partie de l'un ou des plusieurs capteurs en contact avec la peau (106, 108, 110) est prévue sur une surface intérieure de panneau d'un panneau ou de panneaux (52) dans une des régions latérales (6, 8), **caractérisé en ce que** la totalité de l'ensemble de capteurs (104) est prévue dans une des régions latérales (8) et n'est pas prévue dans la sous-bande (14).

2. Soutien-gorge (1) tel que revendiqué dans la revendication 1, dans lequel la région latérale (8) où l'ensemble de capteurs (104) est prévu comprend une poche (54) dans laquelle un module électronique (102) de l'appareil de mesure (100) est situé.

3. Soutien-gorge (1) tel que revendiqué dans la revendication 2, dans lequel la poche (54) est une poche élastiquement résiliente (54).

4. Soutien-gorge (1) tel que revendiqué dans la revendication 2 ou 3, dans lequel la poche (54) est une poche camouflée.

5. Soutien-gorge (1) tel que revendiqué dans l'une quelconque des revendications 2 à 4, dans lequel l'intérieur de la poche (54) est accessible pour donner l'accès au module électronique (102) de l'appareil de mesure (100).

6. Soutien-gorge (1) tel que revendiqué dans la revendication 5, dans lequel le module électronique (102) peut être enlevé de la poche (54).

7. Soutien-gorge (1) tel que revendiqué dans la revendication 5 ou 6, dans lequel l'un ou les plusieurs capteurs (106, 108, 110) sont connectables de façon libérable au module électronique (102).

8. Soutien-gorge tel que revendiqué dans l'une quelconque des revendications 2 à 7, dans lequel la région avant (2) comprend un panneau avant (10a) et la région arrière (4) comprend un panneau arrière (12), et le panneau avant (10a) et/ou le panneau arrière (2) s'étendent dans la région latérale (6, 8), et la poche (54) est prévue sur ou dans le panneau ou les panneaux (52) dans la région latérale (8).

9. Soutien-gorge (1) tel que revendiqué dans l'une quelconque des revendications 2 à 8, dans lequel la région latérale (8) comprend un panneau latéral (52), et la poche (54) est prévue sur ou dans le panneau latéral (52).

10. Soutien-gorge (1) tel que revendiqué dans l'une ou l'autre des revendications 8 ou 9, dans lequel au moins une partie de l'ensemble de capteurs (104) est prévue sur une surface intérieure de panneau et la poche (54) est prévue sur une surface extérieure de panneau.

11. Soutien-gorge (1) tel que revendiqué dans une quelconque revendication précédente, dans lequel l'appareil de mesure (100) est configuré pour réaliser une ou plusieurs des mesures suivantes : électrocardiographie ; bioélectricité ; bioimpédance.

12. Procédé de fabrication d'un soutien-gorge (1) tel que revendiqué dans l'une quelconque des revendications 1 à 11, le procédé comprenant les étapes de : la fourniture d'un soutien-gorge (1) comprenant : une région avant (2) ; une région arrière (4) ; une paire de régions latérales (6, 8) s'étendant entre la région avant (2) et la région arrière (4) ; une sous-bande (14) s'étendant en dessous d'un bord inférieur de la région avant (2), de la région arrière (4) et des régions latérales (6, 8) ; et un appareil de mesure (100) comprenant un ensemble de capteurs (104) comprenant un ou plusieurs capteurs en contact avec la peau (106, 108, 110), dans lequel au moins une partie de l'un ou des plusieurs capteurs en contact avec la peau (106, 108, 110) est prévue sur une surface intérieure de panneau d'un panneau ou de panneaux (52) dans une des régions latérales (6, 8) ; **caractérisé en ce que** la totalité de l'ensemble de capteurs (104) est prévue dans une des régions latérales (8) et n'est pas prévue dans la sous-bande (14).
